# EUROPÄISCHE PATENTANMELDUNG

(11) **EP 0 904 834 A2**
(43) Veröffentlichungstag der Anmeldung: **31.03.1999**
(21) Anmeldenummer: 98117449.3
(22) Anmeldetag: 15.09.1998
(51) Int. Cl.: B01J 20/28, B01D 39/20, B01J 47/00, B01J 37/00

(54) **Adsorptiver Filterkörper**

(30) Priorität: 26.09.1997 DE 19742572
(71) Anmelder: HELSA-WERKE HELMUT SANDLER GmbH & CO. KG, 95482 Gefrees (DE)
(72) Erfinder: Keibel, Thorsten, 95145 Oberkotzau (DE); Degelmann, Walter, 95326 Kulmbach (DE); Krull, Manfred, 95488 Eckersdorf (DE)
(74) Vertreter: LOUIS, PÖHLAU, LOHRENTZ & SEGETH

(57) **Zusammenfassung**

Es wird ein Filterkörper aus miteinander mittels Kleberpartikeln verklebten Adsorberpartikeln und mit Durchgangskanälen beschrieben, die sich durch den Filterkörper erstrecken. Der Filterkörper besteht aus einem Gemisch aus partikel- und/oder kornförmigen Sorbienten und/oder Katalysatoren und/oder Ionenaustauschern und ausgehärteten Kleberpartikeln, die in einem definierten Verhältnis gemischt sind. Der Gewichtsanteil der Kleberpartikel beträgt 2 bis 40 Gew.-%.

## Beschreibung

Die Erfindung betrifft einen Filterkörper aus miteinander verbundenen Adsorberpartikeln.

Adsorptive Filterkörper kommen bspw. als sog. Schüttbettfilter zur Anwendung. Bei solchen Schüttbettfiltern sind die Adsorberpartikel in einem Gehäuse in loser Schüttung vorgesehen. Der Durchströmwiderstand, d.h. der Druckabfall derartiger Schüttbettfilter ist entsprechend groß. Zur Reduktion des Durchströmwiderstandes bzw. des Druckabfalles sind auch adsorptive Filterkörper bekannt, die in einem Extrusionsverfahren hergestellt werden. Die EP 0 492 081 A1 beschreibt einen solchen Filterkörper aus Adsorberpartikeln, der im Extrusionsverfahren realisiert wird. Um die Adsorberpartikel extrudieren zu können, müssen sie mit einem Plastifizierungsmittel vermischt werden. Extrusionsvorrichtungen zur Realisierung solcher extrudierter Filterkörper sind in ihrer Anschaffung teuer. Zur Produktion relativ kleiner Stückzahlen adsorptiver Filterkörper sind solche Extrusionsvorrichtungen nur sehr bedingt bzw. nicht geeignet, weil ihr Anschaffungspreis den Stückpreis der extrudierten Filterkörper zu stark beeinflussen würde.

Geruchsfilter zur Innenluftreinigung in Kraftfahrzeugen bestehen üblicherweise aus einem Kunststoffschaummaterial, an welchem mit Hilfe eines Klebers Aktivkohle fixiert ist. Der Strömungswiderstand solcher Geruchsfilter soll bei einer Strömungsgeschwindigkeit von 130 cm/sek. < 320 Pa sein. Die gesamte adsorbierte Masse soll bei einer Strömungsgeschwindigkeit von 70 cm/sek größer 15 g n-Butan bei 80 ppmV und 35% relativer Feuchte sein. Der Sofortdurchbruch bei einer Strömungsgeschwindigkeit von 70 cm/sek und 250 ppmV n-Butan und einer Filtertiefe von 4 cm soll höchstens 0,1 % betragen.

Durch das Kunststoffschaummaterial bekannter Geruchsfilter wird eine irreguläre Struktur aufgebaut. Eine derartige irreguläre Struktur führt zu einem hohen Druckabfall.

Kraftfahrzeuge besitzen ein genau berechnetes Energieverteilungssystem, wobei für Heizung, Lüftung bzw. Klima nur eine begrenzte Energiemenge zur Verfügung steht. Ein großer Druckabfall bekannter Filterkörper der oben genannten Art bedingt einen entsprechend großdimensionierten Lüftermotor und folglich einen relativ großen Energieverbrauch im Kraftfahrzeug. Ein solcher großdimensionierter Lüftermotor bedingt außerdem eine unerwünschte Geräuschentwicklung im Fahrzeug.

Die gesamte adsorbierte Masse steht mit der Standzeit eines Filters in engem Zusammenhang. Ist die Kapazität eines Filters erschöpft, so ist es erforderlich, das Filter zu wechseln. Höhere Kapazitäten bzw. längere Standzeiten bekannter Filter sind nur mit eine relativ großen Aktivkohle-Menge zu erzielen. Das bedeutet einerseits eine entsprechende Gewichtszunahme im Fahrzeug und andererseits einen weiter erhöhten Druckabfall mit all den oben beschriebenen Mängeln.

Eine besonders kritische Aufmerksamkeit erlangt diese Tatsache durch die Forderung nach immer kleineren Anströmflächen der Filterkörper. Das resultiert aus der immer kompakteren Bauweise der Fahrzeuge, d.h. den Einzelkomponenten steht nur ein immer kleinerer Raum zur Verfügung.

Bei einem Einsatz von Gebläsen mit einer relativ geringen Leistung wird in Fahrzeugen oftmals eine zusätzliche Bypaßleitung vorgesehen, um beim Defrosten den maximal nötigen Volumenstrom durch einen Kurzschluß des Filters mit Hilfe der Bypaßleitung zu erzielen. Durch eine solche Ausbildung steigt zwar der Komfort in einem Fahrzeug, gleichzeitig steigt jedoch die Anzahl der Einzelteile, was sich u.a. auch auf den Wartungsaufwand auswirkt. Um den Wartungsaufwand in vernünftigen Grenzen zu halten, ist es folglich erforderlich, in das Fahrzeug möglichst wartungsfreie Einzelkomponenten zu integrieren. Derartige wartungsfreie Langzeitfilter können auch an schwer zugänglichen Stellen im Fahrzeug vorgesehen werden.

Der Erfindung liegt die Aufgabe zugrunde, einen Filterkörper zu schaffen, der in einfacher Weise preisgünstig realisierbar ist, wobei der Durchströmwiderstand bzw. der Druckabfall ähnlich klein ist wie der eines extrudierten adsorptiven Filterkörpers, und den Filterkörper so zu gestalten, daß er bei einem vergleichsweise kleinen Einbauvolumen eine hohe Sorptionsleistung aufweist, wobei außerdem die Standzeit des Filterkörpers erhöht ist.
Diese Aufgabe wird bei einem Filterkörper der eingangs genannten Art erfindungsgemäß dadurch gelöst, daß er aus einem Gemisch aus partikel- und/oder kornförmigen Sorbienten und/oder Katalysatoren und/oder Ionenaustauschern und ausgehärteten Kleberpartikeln besteht, die in einem definierten Verhältnis derart gemischt sind, daß der Gewichtsanteil der Kleberpartikel zwischen 2 und 40 Gew.-% beträgt.

Der erfindungsgemäße Filterkörper eignet sich zur Sorption von Schadstoffen sowohl aus der Gasphase als auch aus der Flüssigphase. Er ist derartig ausgelegt, daß mit ihm ein breites Spektrum von Geruchs- und/oder Schadstoffen in der Weise sorbiert werden kann, daß die Sorption zeitlich sehr schnell bei Kontaktzeiten im Bereich von 50 msek erfolgt und die Desorption nur allmählich und stark verzögert eintritt. Eine kurzfristig hohe Konzentration an Geruchs- bzw. Schadstoffen wird somit durch den erfindungsgemäßen Filterkörper gepuffert und bleibt in vorteilhafter Weise unterhalb einer vorgegebenen Geruchs- bzw. Beeinträchtigungsschwelle.

Bei dem erfindungsgemäßen Filterkörper können die Sorbienten von Aktivkohle und/oder Zeolithen und/oder Silicagel und/oder Aluminiumoxid gebildet sein. Selbstverständlich sind auch andere an sich bekannte Sorbienten anwendbar. Die Sorbienten besitzen vorzugsweise eine Partikel- bzw. Korngröße zwischen 10 µm und 4 mm.

Die Kleberpartikel des erfindungsgemäßen Filterkörpers bestehen vorzugsweise aus thermoplastischem Material; es ist auch möglich, daß die Kleberpartikel aus thermoplastischem Material bestehen, das nach der Aktivierung zu einem Duroplasten vernetzt. Die Kleberpartikel besitzen vorzugsweise eine Korngröße zwischen 1 µm und 2 mm, vorzugsweise zwischen 80 und 200 µm. Der erfindungsgemäße Filterkörper kann in vorteilhafter Weise mit einer dem jeweiligen Einsatzzweck entsprechenden, beliebigen Geometrie gestaltet werden bzw. gestaltet sein. Durch das Gemisch aus partikel- und/oder kornförmigen Sorbienten und/oder Katalysatoren und/oder Ionenaustauschern und ausgehärteten Kleberpartikeln ergibt sich ein Filterkörper mit einem festen Verbund der Partikel bzw. Körner, so daß gegebenenfalls auf eine Abdeckung des Filterkörpers, die ein Ausrieseln der besagten Partikel bzw. Körner verhindert, verzichtet werden kann. Das stellt einen weiteren Vorteil des erfindungsgemäßen Filterkörpers dar.

Das Verhältnis von lichter Gesamtfläche der Durchgangskanäle zu Anströmfläche des Filterkörpers kann zwischen 0,1 und 0,9 betragen. Dieses Verhältnis wird den Anforderungen bezüglich Strömungswiderstand, der gesamten adsorbierten Masse bzw. Sofortdurchbruch entsprechend eingestellt.

Als besonders vorteilhaft hat es sich erwiesen, wenn der Filterkörper mindestens zwei Filterkörper-Lagen aufweist, die durch einen Spalt voneinander getrennt vorgesehen sind. Dabei fluchten die Außenränder der Filterkörper-Lagen vorzugsweise miteinander. Weist bspw. ein monolithischer Filterkörper, d.h. ein einlagiger Filterkörper mit einer Filtertiefe bzw. Bauhöhe von 4 cm einen Sofortdurchbruch in der Größenordnung von 6 % auf, so ergibt sich bei dem "gleichen monolithischen" Filterkörper bei einmaliger Teilung desselben senkrecht zu den Durchgangskanälen überraschenderweise ein Sofortdurchbruch in der Größenordnung von 4 % und bei gevierteltem Filterkörper ein Sofortdurchbruch in der Größenordnung von 1,5 %.

Beispielsweise besitzt der erfindungsgemäße Filterkörper bei einer Filtertiefe von 3 cm, einer Strömungsgeschwindigkeit von 70 cm/sek, einer Konzentration von 250 ppmV n-Butan und einer Temperatur von 23°C einen Sofortdurchbruch zwischen 1‰ und 10 %. Wird der besagte Filterkörper in der zuletzt genannten Art senkrecht zur Strömungsrichtung zerteilt und werden die beiden Teile voneinander um ca. 1 mm beabstandet, so wird der Sofortdurchbruch um ca. 30 - 70 % ,vorzugsweise 50 % reduziert. Die Reduzierung des Sofortdurchbruchs kann durch wiederholte Weiterteilung des Filterkörpers und geeignete Beabstandung der auf diese Weise hergestellten Filterkörper-Lagen in vorteilhafter Weise wunschgemäß weiter reduziert werden. Die spezifische Sorptionskapazität des erfindungsgemäßen Filterkörpers für n-Butan bei 80 ppmV und 23°C pro 1000 cm³ Filterkörper-Volumen kann ca. 24 g betragen.

Erfindungsgemäß kann der Filterkörper mit einem Vorfilter kombiniert sein. Dieser Vorfilter kann in vorteilhafter Weise dazu vorgesehen sein, den Sofortdurchbruch bspw. von n-Butan bei einer Strömungsgeschwindigkeit von 70 cm/sek, einer Konzentration von 250 ppmV n-Butan und einer Temperatur von 23°C bei 35 % relativer Feuchte um 30 bis 99 %, vorzugsweise um größenordnungsmäßig 70 %, zu reduzieren.

Der Vorfilter kann zum Schutz des Filterkörpers vor Ruß und/oder Staubpartikeln und/oder hochsiedenden Substanzen wie Toluol, Xylol usw. vorgesehen sein. Derartige hochsiedende Substanzen sind bekanntermaßen nur sehr schwer, d.h. nur bei hohen Temperaturen, zu desorbieren. Hier kann mit dem Vorfilter in einfacher Weise Abhilfe geschaffen werden.

Bei dem erfindungsgemäßen Filterkörper kann der Vorfilter zum Abtrennen von Allergenen und/oder Pollen und/oder Bakterien und/oder Krankheitserregern vorgesehen sein. Der Vorfilter kann auch mit Katalysatoren und/oder mit chemisorptiv aktiven Sorbienten ausgerüstet sein. Ein Vorfilter, der Wasser sorbiert oder chemisch bindet, verbessert die Adsorptionsleistung des Hauptfilters, d.h. des Filterkörpers um 10 - 50%.

Es hat sich gezeigt, daß der erfindungsgemäße Filterkörper nach einer Temperaturbehandlung im Temperaturbereich von 50°C bis 250°C, vorzugsweise im Temperaturbereich von 80°C bis 120°C, desorbierbar ist. Ebenso kann auf diese Weise das gebundene Wasser aus dem Vorfilter ausgetrieben werden. Eine solche Desorption kann in zeitlich regelmäßigen oder in zeitlich unregelmäßigen Abständen erfolgen. Die Desorption kann bspw. auch mit Wasserdampf oder mit anderen aus der Sorptionstechnik bekannten Verdrängungsmitteln erfolgen. Besonders vorteilhaft ist es, den ausgetriebenen Wasserdampf aus dem Vorfilter als Verdrängungsmittel für den Hauptfilter zu nutzen.

Erfindungsgemäß kann der erfindungsgemäße Filterkörper zur Reinigung der Zu- und Innenluft in einem Kraftfahrzeug Verwendung finden. In Kombination mit einem Vorfilter der oben erwähnten Art ergibt sich der Vorteil, daß der erfindungsgemäße Filterkörper über die gesamte Lebensdauer eines Fahrzeuges einsetzbar ist, d.h. einen sog. "Long-Time-Filter" bildet. Ein solcher "Long-Time-Filter" kann in vorteilhafter Weise auch an schwer zugänglichen Stellen eines Fahrzeuges eingebaut werden.

Eine andere erfindungsgemäße Verwendung des oben abgehandelten Filterkörpers ist das Gebiet der Atemschutzeinrichtungen. Desweiteren kann er zur Reinigung der Zu- und Abluft eines Gebäudes Verwendung finden. Durch die realisierbare ausgezeichnete Steifigkeit des erfindungsgemäßen Filterkörpers ist es möglich, bspw. Gebäude-Flächenelemente zu verstellen, die z.B. eine Grundfläche von 0,3 m² besitzen. Außerdem kann der erfindungsgemäße Filterkörper zur Reinigung der Luft in geschlossenen Innenräumen verwendet werden.

Außerdem kann der erfindungsgemäße Filterkörper bei einer Wasserreinigungseinrichtung oder als Katalysatoreinrichtung in der chemischen Industrie Verwendung finden. Desweiteren ist es möglich, den Filterkörper zur gezielten Abgabe von Duftstoffen und/oder Pheromonen und/oder Insektiziden und/oder Pharmazeutika und/oder anderer an sich bekannter Wirkstoffe zu verwenden.

Nachfolgend wird der Erfindungsgegenstand anhand einiger Ausführungsbeispiele weiter erläutert:
1.1 Vermessen wurde ein Aktivkohlefilter mit 28 Kanälen/cm² Gesamt-Anströmgrundfläche (=180 Durchgangskanäle pro inch²) und einer Filtertiefe von 4 cm mit folgenden Parametern (bei 23°C und 35 % relativer Feuchte):
- Sofortdurchbruch:: 2 % bei 250 ppmV n-Butan und einer Durchströmgeschwindigkeit von 70 cm/sek
- gesamte adsorbierte Masse:: 55 g n-Butan bei 80 ppmV und einer Durchströmgeschwindigkeit von 70 cm/sek
- Strömungswiderstand:: 210 Pa bei 130 cm/sek Durchströmgeschwindigkeit

1.2 Sorptiver Vorfilter: (bei 23°C und 35% relativer Feuchte)
- Sofortdurchbruch :: 12,5 % bei 250 ppmV n-Butan und 70 cm/sek Durchströmgeschwindigkeit
- gesamte adsorbierte Masse:: 5,1 g n-Butan bei 80 ppmV und 70 cm/sek Durchströmgeschwindigkeit
- Strömungswiderstand:: 61 Pa bei 130 cm/sek Durchströmgeschwindigkeit

1.3 Gesamtsystem (aus Filterkörper gemäß 1.1 und Vorfilter gemäß 1.2; bei 23°C und 35 % relativer Feuchte):
- Sofortdurchbruch:: <0,1 % bei 250 ppmV n-Butan und 70 cm/sek
- gesamtads. Masse:: 60 g n-Butan bei 80 ppmV und 70 cm/sek
- Strömungswiderstand:: 290 Pa bei 130 cm/sek.

2. Sofortdurchbruch bei Filterkörpern mit einer Filtertiefe von 4 cm, 180 Durchgangskanälen pro Quadratinch und einem Kleberanteil von 10 Gew.-%(bei 23°C, 35 % relativer Feuchte, 250 ppmV n-Butan, 70 cm/sek):

| Kohle | Körnung | Oberfläche | Sofortdurchbruch |
|---|---|---|---|
| Aktivkohle (aus Kokosnuß) | 0,2-05 mm | 1150m²/g(BET) | 5,3 % |
| Pulverkohle | 8-15 µm | 1600 m²/g (Jodzahl) | 16 % |

3. Sofortdurchbrüche bei Filterkörpern mit einer Filtertiefe von insgesamt 4 cm (bei 23°C und 35 % relativer Feuchte):
6 % bei "monolithischem" (-d.h.einlagigem) Filterkörper
4 % bei einmal geteiltem Filterkörper
1,5 % bei viergeteiltem (d.h. in vier Filterkörper-Lagen

Zur Realisierung des erfindungsgemäßen Filterkörpers können die mit einem geeigneten Kleber benetzten Adsorberpartikel oder die Adsorberpartikel gemeinsam mit Kleberpartikeln in eine Form eingebracht werden, die mit die Durchgangskanäle des Filterkörpers bildenden länglichen Elementen wie Nadeln o.dgl. versehen ist. Die besagte Form wird mit den mit Kleber benetzten Adsorberpartikeln bzw. mit Adsorberpartikeln und Kleberpartikeln befüllt. Es erfolgt dann mit Hilfe des Klebers eine Verbindung der Adsorberpartikel miteinander zum Filterkörper. Danach werden die länglichen Elemente aus der Form entfernt, wodurch sich die Durchgangskanäle im Filterkörper ergeben. Anschließend kann der Filterkörper aus der Form entnommen werden.

Die länglichen Elemente der besagten Form können von Nadeln gebildet sein, die sich geradlinig durch den Filterkörper erstrecken. Ein solchermaßen ausgebildeter Filterkörper ist relativ einfach realisierbar.

Die Durchgangskanäle sind zueinander parallel vorgesehen, um alle länglichen Elemente aus der oben erwähnten Form gleichzeitig entfernen zu können und hierdurch in einem Arbeitsschritt alle Durchgangskanäle gleichzeitig auszubilden.

Der erfindungsgemäße Filterkörper kann von einem Gehäusemantel umgeben sein. Dieser Gehäusemantel kann Teil der erwähnten Form sein, die mit den länglichen Elementen zur Realisierung der Durchgangskanäle versehen ist und die mit den mit Kleber benetzten Adsorberpartikeln bzw. mit Adsorberpartikeln und Kleberpartikeln befüllt wird, um nach der Vernetzung des Klebers den Filterkörper zu bilden.

Bei einem solchen Filterkörper der zuletzt genannten Art kann der Gehäusemantel aus einem Kunststoffmaterial bestehen. Eine andere Möglichkeit besteht darin, daß der Gehäusemantel des erfindungsgemäßen Filterkörpers aus einem textilen Material besteht. Ein solches textiles Material weist eine solche Maschenweite auf, daß Adsorberpartikel, die sich gegebenenfalls vom kompakten und mit Durchgangskanälen ausgebildeten Filterkörper ablösen, daran gehindert werden, aus dem Gehäusemantel auszutreten.

Der erfindungsgemäße Filterkörper kann an seiner Grundfläche und an seiner Deckfläche jeweils mit einem Deckel versehen sein, der mit dem Gehäusemantel verbunden ist, wobei sich die Durchgangskanäle durch die beiden Deckel hindurcherstrecken. Diese Deckel bilden gemeinsam mit dem Gehäusemantel insbes. dann einen guten Schutz gegen mechanische Einwirkungen von außen,wenn die beiden Deckel - wie der Gehäusemantel - aus einem Kunststoffmaterial bestehen. Desgleichen ist es möglich, daß die beiden Deckel aus einem textilen Material bestehen. Bei einer Ausbildung der zuletzt genannten Art gilt für die Maschenweite des textilen Materials für die beiden Deckel das oben in Bezug zum Gehäusemantel Gesagte entsprechend.

Weitere Einzelheiten, Merkmale und Vorteile ergeben sich aus der nachfolgenden Beschreibung von in der Zeichnung schematisch dargestellten Ausführungsbeispielen des erfindungsgemäßen Filterkörpers. Es zeigen:
- Fig. 1: längsgeschnitten eine erste Ausführungsform des Filterkörpers,
- Fig. 2: in einer der Fig. 1 ähnlichen Schnittdarstellung eine zweite Ausbildung des Filterkörpers,
- Fig. 3: eine dritte Ausführungsform des Filterkörpers in einer den Figuren 1 und 2 entsprechenden Schnittdarstellung, und
- Fig. 4: eine vierte Ausführungsform des Filterkörpers in einer den Figuren 1 bis 3 ähnlichen Längsschnitt-Darstellung.

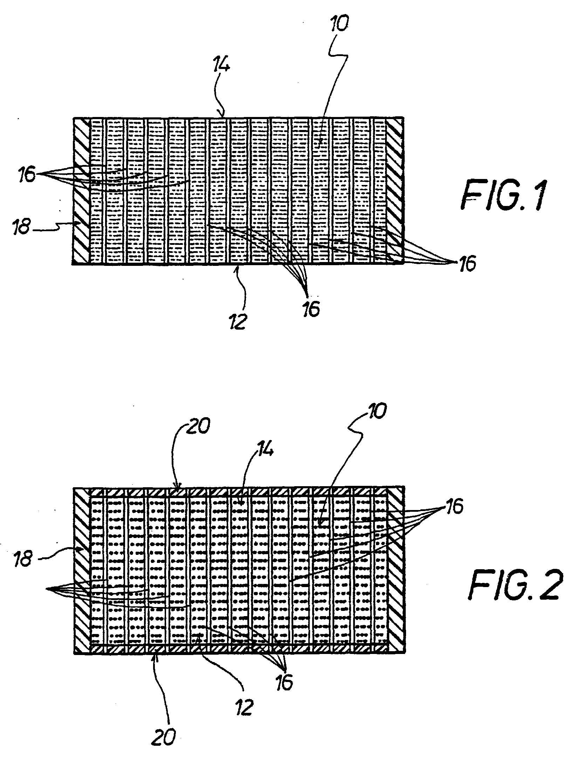

Fig. 1 zeigt längsgeschnitten eine erste Ausführungsform des Filterkörpers 10 aus miteinander mittels eines Klebers verbundenen Adsorberpartikeln. Der Filterkörper 10 weist eine Grundfläche 12 und eine Deckfläche 14 auf, zwischen welchen sich durch den Filterkörper 10 Durchgangskanäle 16 hindurcherstrecken. Die Durchgangskanäle 16 verlaufen geradlinig und voneinander beabstandet zueinander parallel.

Der Filterkörper 10 ist von einem Gehäusemantel 18 umgeben, der mit dem Filterkörper 10 fest verbunden ist. Der Gehäusemantel 18 besteht aus einem Kunststoffmaterial.

Fig. 2 zeigt eine zweite Ausbildung des Filterkörpers 10, die sich von der in Fig. 1 gezeichneten Ausführungsform des Filterkörpers 10 insbes. dadurch unterscheidet, daß der Filterkörper 10 an seiner Grundfläche 12 und an seiner Deckelfläche 14 jeweils mit einem Deckel 20 aus einem Kunststoffmaterial versehen ist. Die beiden Deckel 20 sind mit dem Gehäusemantel 18 aus einem Kunststoffmaterial fest verbunden. Die Deckel 20 und der Gehäusemantel 18 können aus dem gleichen Kunststoffmaterial bestehen und miteinander einstückig verbunden sein. Die Durchgangskanäle 16 erstrecken sich bei dieser Ausbildung des Filterkörpers 10 nicht nur durch den Filterkörper aus miteinander mittels eines Klebers verbundenen Adsorberpartikeln hindurch, sondern außerdem auch durch die beiden Deckel 20 des Filterkörpers 10.

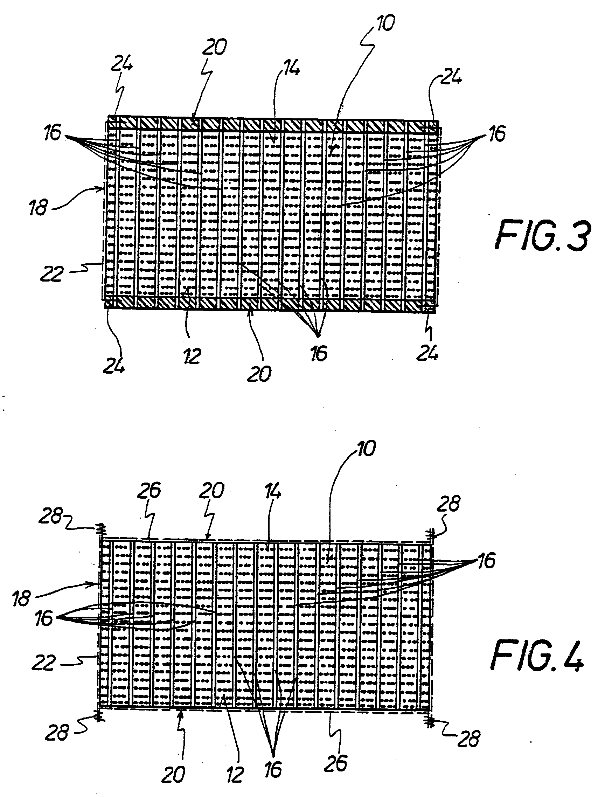

Fig. 3 zeigt eine dritte Ausbildung des Filterkörpers 10 aus miteinander mittels eines Klebers verbundenen Adsorberpartikeln, durch den sich Durchgangskanäle 16 voneinander beabstandet und zueinander parallel verlaufend geradlinig hindurcherstrecken. Die Grundfläche 12 und die Deckfläche 14 des Filterkörpers 10 sind jeweils mit einem Deckel 20 aus Kunststoffmaterial fest verbunden. Die Durchgangskanäle 16 erstrecken sich nicht nur durch den Filterkörper 10 sondern auch durch die beiden Deckel 20 hindurch.

Der Filterkörper 10 gemäß Fig. 3 unterscheidet sich von dem in Fig. 2 gezeichneten Filterkörper 10 insbes. dadurch, daß der Gehäusemantel 18 beim Filterkörper 10 gemäß Fig. 3 von einem textilen Material 22 gebildet ist, das mit seinen Rändern 24 in die beiden Deckel 20 aus Kunststoffmaterial eingebettet ist, um eine mechanisch feste Verbindung zu bewerkstelligen.

Fig. 4 zeigt eine Ausbildung des Filterkörpers 10 aus miteinander mittels eines Klebers verbundenen Adsorberpartikeln, wobei sowohl der Gehäusemantel 18 als auch die beiden Deckel 20 jeweils von einem textilen Material 22 und 26 gebildet sind. Die Deckel 20 aus textilem Material 26 weisen eine bestimmte Gasdurchlässigkeit auf. Der Filterkörper 10 ist mit geradlinig verlaufenden Durchgangskanälen 16 ausgebildet, die voneinander beabstandet sind und die zueinander parallel verlaufen. Die Durchgangskanäle 16 erstrecken sich durch den Filterkörper 10 hindurch. Der Gehäusemantel 18 aus textilem Material 22 und die beiden Deckel 20 aus textilem Material 26 sind miteinander vernäht. Die Verbindungsnähte sind mit der Bezugsziffer 28 bezeichnet.

## Patentansprüche

1. Filterkörper aus miteinander mittels Kleberpartikeln verklebten Adsorberpartikeln und mit voneinander beabstandeten Durchgangskanälen, die sich durch den Filterkörper erstrecken, wobei der Filterkörper aus einem Gemisch aus partikel- und/oder kornförmigen Sorbienten und/oder Katalysatoren und/oder Ionenaustauschern und ausgehärteten Kleberpartikeln besteht, die in einem definierten Verhältnis derart gemischt sind, daß der Gewichtsanteil der Kleberpartikel zwischen 2 und 40 Gew.-% beträgt.

2. Filterkörper nach Anspruch 1,
**dadurch gekennzeichnet,**
daß die Sorbienten von Aktivkohle und/oder Zeolithen und/oder Silicagel und/oder Aluminiumoxid gebildet sind.

3. Filterkörper nach Anspruch 2,
**dadurch gekennzeichnet,**
daß die Sorbienten eine Korngröße zwischen 10 µm und 4 mm besitzen.

4. Filterkörper nach Anspruch 1,
**dadurch gekennzeichnet,**
daß die Kleberpartikel aus thermoplastischem Material bestehen.

5. Filterkörper nach Anspruch 4,
**dadurch gekennzeichnet,**
daß die Kleberpartikel aus thermoplastischem Material bestehen, die nach der Aktivierung zu Duroplasten vernetzen.

6. Filterkörper nach Anspruch 4 oder 5,
**dadurch gekennzeichnet,**
daß die Kleberpartikel eine Korngröße zwischen 1 µm und 2 mm, vorzugsweise zwischen 80 und 200 µm, besitzen.

7. Filterkörper nach einem der vorhergehenden Ansprüche,
**dadurch gekennzeichnet,**
daß das Verhältnis von lichter Gesamtfläche der Durchgangskanäle zu Anströmfläche des Filterkörpers zwischen 0,1 und 0,9 beträgt.

8. Filterkörper nach einem der vorhergehenden Ansprüche,
**dadurch gekennzeichnet,**
daß der Filterkörper mindestens zwei Filterkörper-Lagen aufweist, die voneinander durch einen Spalt beabstandet zueinander benachbart vorgesehen sind.

9. Filterkörper nach Anspruch 8,
**dadurch gekennzeichnet,**
daß die Durchgangskanäle der Filterkörper nacheinander durchströmt werden.

10. Filterkörper nach einem der vorhergehenden Ansprüche,
**dadurch gekennzeichnet,**
daß der Filterkörper mit einem Vorfilter kombiniert ist.

11. Filterkörper nach Anspruch 10,
**dadurch gekennzeichnet,**
daß der Vorfilter aus mehreren Schichten besteht.

12. Filterkörper nach Anspruch 10 oder 11,
**dadurch gekennzeichnet,**
daß der Vorfilter zum Schutz des Filterkörpers vor Ruß- und/oder Staubpartikeln und/oder hochsiedenden Substanzen vorgesehen ist.

13. Filterkörper nach Anspruch 10 oder 11,
**dadurch gekennzeichnet,**
daß der Vorfilter zum Abtrennen von Allergenen und/oder Pollen und/oder Bakterien und/oder Krankheitserregern vorgesehen ist.

14. Filterkörper nach Anspruch 10 oder 11,
**dadurch gekennzeichnet,**
daß der Vorfilter mit Katalysatoren und/oder mit chemisorptiv aktiven Sorbienten ausgerüstet ist.

15. Filterkörper nach Ansprüchen 10 oder 11,
**dadurch gekennzeichnet,**
daß der Vorfilter zum Schutz des Filterkörpers vor Wasserdampf vorgesehen ist.

16. Filterkörper nach einem der Ansprüche 1 bis 15,
**dadurch gekennzeichnet,**
daß der Filterkörper nach einer Temperaturbehandlung im Temperaturbereich von 50°C bis 250°C, vorzugsweise von 80°C bis 120°C, desorbiert ist.

17. Filterkörper nach einem der Ansprüche 1 bis 16,
**dadurch gekennzeichnet,**
daß der Filterkörper nach einer Behandlung mit Wasserdampf oder anderen aus der Sorptionstechnik bekannten Verdrängungsmitteln desorbiert ist.

18. Filterkörper nach einem der Ansprüche 1 bis 17,
**dadurch gekennzeichnet,**
daß der Wasserdampf zur Desorption des Filterkörpers aus dem Vorfilter stammt.

19. Verwendung eines Filterkörpers nach einem der Ansprüche 1 bis 18 zur Reinigung der Zu- und Innenluft in einem Kraftfahrzeug.

20. Verwendung eines Filterkörpers nach einem der Ansprüche 1 bis 18 bei einer Atemschutzeinrichtung.

21. Verwendung eines Filterkörpers nach einem der Ansprüche 1 bis 18 zur Reinigung der Zu- und Abluft eines Gebäudes.

22. Verwendung eines Filterkörpers nach einem der Ansprüche 1 bis 18 zur Reinigung der Luft in geschlossenen Innenräumen.

23. Verwendung eines Filterkörpers nach einem der Ansprüche 1 bis 18 bei einer Wasserreinigungseinrichtung.

24. Verwendung eines Filterkörpers nach einem der Ansprüche 1 bis 18 als Katalysatoreinrichtung in der chemischen Industrie.

25. Verwendung eines Filterkörpers nach einem der Ansprüche 1 bis 18 zur gezielten Abgabe von Duftstoffen und/oder Pheromonen und/oder Insektiziden und/oder Pharmazeutika und/oder anderen an sich bekannten Wirkstoffen.
